(19) Europäisches Patentamt
European Patent Office
Office européen des brevets

(11) EP 4 755 874 A1

(12) **EUROPEAN PATENT APPLICATION**
published in accordance with Art. 153(4) EPC

(43) Date of publication:
**10.06.2026 Bulletin 2026/24**

(21) Application number: **24849224.1**

(22) Date of filing: **31.07.2024**

(51) International Patent Classification (IPC):
***C07C 381/12*** *(2006.01)* ***C07F 11/00*** *(2006.01)*
***G03F 7/004*** *(2006.01)* ***G03F 7/038*** *(2006.01)*

(52) Cooperative Patent Classification (CPC):
**C07C 381/12; C07F 11/00; G03F 7/004; G03F 7/038**

(86) International application number:
**PCT/JP2024/027411**

(87) International publication number:
**WO 2025/028579 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.08.2023 JP 2023127352**

(71) Applicant: **TOKYO OHKA KOGYO CO., LTD.**
**Kawasaki-shi, Kanagawa 211 0012 (JP)**

(72) Inventors:
- **NODA, Kunihiro**
  **Kawasaki-shi, Kanagawa 211-0012 (JP)**
- **INARI, Takatoshi**
  **Kawasaki-shi, Kanagawa 211-0012 (JP)**
- **NISHIZAWA, Akito**
  **Kawasaki-shi, Kanagawa 211-0012 (JP)**
- **NAGATA, Masaya**
  **Kawasaki-shi, Kanagawa 211-0012 (JP)**

(74) Representative: **Plasseraud IP**
**104 Rue de Richelieu**
**CS92104**
**75080 Paris Cedex 02 (FR)**

(54) **METHOD FOR PRODUCING METATUNGSTATE COMPOUND, METATUNGSTATE COMPOUND FOR RESISTS, AND RESIST MATERIAL**

(57) The present invention provides a method for selectively producing a metatungstic acid salt compound containing 5 onium cations or onium dications by performing a salt exchange reaction of a metatungstic acid salt in an acidic solution having a pH of less than 3, a novel metatungstic acid salt compound for resists produced by such a production method, and a resist material containing the same.

EP 4 755 874 A1

## Description

### Technical Field

The present invention relates to a method for producing metatungstate compound, metatungstate compound for resists, and resist material.

### Background Art

Ammonium metatungstate has been widely used in the production of metallic tungsten, alloy steels, flame-retardant fabrics, and the like, and in the manufacture of petrochemical catalysts and the like.

In recent years, resist-related compositions containing a metatungstic acid salt have been reported. For example, PTL 1 discloses a resist underlayer film-forming composition containing a metatungstic acid salt (e.g., an alkali metal salt or an ammonium salt) and a polysiloxane or the like. PTL 2 discloses a composition containing an ammonium metatungstate salt and a silane or a polysiloxane, which is to be applied to a resist pattern for performing pattern reversal by utilizing a difference in etching rate.

### Citation List

### Patent Literature

PTL 1: U.S. Patent No. 9725618
PTL 2: U.S. Patent No. 10558119

### Summary of Invention

### Technical Problem

An object of the present invention is to provide a method for selectively producing a metatungstic acid salt compound in which the cation moiety of a metatungstic acid salt is substituted with a predetermined number of predetermined counter cations. Also provided are a novel metatungstic acid salt compound for resists, and a novel resist material containing such a metatungstic acid salt compound for resists.

### Solution to Problem

As a result of extensive studies conducted to address the above problems, the inventors of the present invention have found that a metatungstic acid salt compound containing 5 onium cations or onium dications can be selectively produced by performing a salt exchange reaction of a metatungstic acid salt in an acidic solution having a pH of less than 3, and have thus completed the present invention.

That is, the present invention relates to the following inventions.

<1> A method for producing a metatungstic acid salt compound represented by General Formula (I-1) or (I-2), the method including a step of performing salt exchange in an acidic solution having a pH of less than 3.

$$(A^{+})_5\cdot[H_3W_{12}O_{40}]^{5-} \qquad (I\text{-}1)$$

$$(A'^{2+})_5\cdot([H_3W_{12}O_{40}]^{5-})_2 \qquad (I\text{-}2)$$

[in Formulae (I-1) and (I-2),

each $A^{+}$ independently represents an onium cation; and
each $A'^{2+}$ independently represents an onium dication.]

<2> The method for producing a metatungstic acid salt compound according to <1>, wherein the onium cation is one or more selected from the group consisting of an organic sulfonium cation, an organic iodonium cation, and an organic

ammonium cation.

<3> The method for producing a metatungstic acid salt compound according to <1>, wherein the acidic solution having a pH of less than 3 contains an inorganic acid, an organic acid having a carboxyl group, or an organic acid having a sulfo group.

<4> The method for producing a metatungstic acid salt compound according to <1>, wherein the salt exchange is salt exchange between a metatungstic acid salt represented by General Formula (II) and an onium salt represented by General Formula (III-1) or (III-2).

$$(B^+)_6 \cdot [H_2W_{12}O_{40}]^{6-} \qquad (II)$$

$$A^+ \cdot X^- \qquad (III\text{-}1)$$

$$A'^{2+} \cdot (X^-)_2 \qquad (III\text{-}2)$$

[in Formula (II),

each $B^+$ independently represents $H^+$, a metal ion, or an ammonium ion;
in Formulae (III-1) and (III-2),
$A^+$ represents an onium cation;
$A'^{2+}$ represents an onium dication; and
$X^-$ represents a monovalent counter anion.]

<5> The method for producing a metatungstic acid salt compound according to <4>, wherein the monovalent counter anion is $Br^-$, $Cl^-$, $I^-$, $Br^{3-}$, $OH^-$, $HSO_4^-$, $CF_3SO_3^-$, $C_2F_5SO_3^-$, $C_3F_7SO_3^-$, $C_4F_9SO_3^-$, p-$CH_3(C_6H_4)SO_3^-$, $ClSO_3^-$, $ClO_4^-$, $NO_3^-$, $(C_6F_5)_4B^-$, $BF_4^-$, $PF_6^-$, $SbF_6^-$, $AlCl_4^-$, $BiF_6^-$, $AsF_6^-$, $(CF_3SO_2)_2N^-$, or $(FSO_2)_2N^-$.

<6> A metatungstic acid salt compound for resists, the metatungstic acid salt compound being represented by General Formula (I-1) or (I-2).

$$(A^+)_5 \cdot [H_3W_{12}O_{40}]^{5-} \qquad (I\text{-}1)$$

$$(A'^{2+})_5 \cdot ([H_3W_{12}O_{40}]^{5-})_2 \qquad (I\text{-}2)$$

[in Formulae (I-1) and (I-2),

each $A^+$ independently represents an onium cation; and
each $A'^{2+}$ independently represents an onium dication.]

<7> A resist material including the metatungstic acid salt for resists according to <6>.

<8> The resist material according to <7>, further including a solvent.

<9> The resist material according to <7> or <8>, for electronic use or for EUV use.

<10> A patterned structure formed using the resist material according to <7> or <8>.

## Advantageous Effects of Invention

According to the present invention, a metatungstic acid salt compound containing 5 onium cations or onium dications can be selectively produced. The metatungstic acid salt compound has improved actinic radiation sensitivity and/or radiation sensitivity, and can be suitably used as a metatungstic acid salt compound for resists. Furthermore, since the metatungstic acid salt compound selectively produced by the production method according to the present invention has a certain degree of uniformity, when the compound is contained in a resist material, a novel resist material with a small change in pattern dimension (CD: critical dimension) can be provided.

## Description of Embodiments

Preferred embodiments of the present invention will be described in detail below. However, the present invention is not limited to the following embodiments.

The term "halogen atom", as used herein, refers to a fluorine atom, a chlorine atom, a bromine atom, or an iodine atom.

In the present description, for example, the term "$C_{1-6}$" or the like means the number of carbon atoms of a group

serving as a mother nucleus.

The term "$C_1$-$_{18}$ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "$C_1$-$_{18}$ hydrocarbylene group" may be replaced by -O-, -S-, -C(=O)-, -COO-, -OCO-, -CONH-, -NHCO-, -NH(C=O)O-, -S-, - SO-, or -$SO_2$- (provided that adjacent divalent carbon atoms are not replaced at the same time).

The "$C_1$-$_{18}$ hydrocarbylene group" is, for example, but not limited to, a "$C_1$-$_{18}$ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "$C_{6-18}$ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "$C_1$-$_{18}$ alkyl group", as used herein, refers to a linear or branched alkyl group with 1 to 18 carbon atoms.

Furthermore, a divalent carbon atom at any position excluding the terminals contained in the "$C_1$-$_{18}$ alkyl group" may be replaced by -O-, -C(=O)-, -C(=O)O-, -OCO-, -CONH-, -NHCO-, -NH(C=O)O-, -S-, or -$SO_2$-. However, adjacent divalent carbon atoms are not replaced at the same time.

The "$C_1$-$_{18}$ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "$C_1$-$_{18}$ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -$SO_2$- is, for example, but not limited to, a 2-methoxyethoxymethyl group, an ethoxycarbonylmethyl group, or the like.

The term "$C_1$-$_{18}$ haloalkyl group", as used herein, refers to a group in which one or more hydrogen atoms of the "$C_1$-$_{18}$ alkyl group" are substituted by a halogen atom.

The "$C_1$-$_{18}$ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "$C_{2-18}$ alkenyl group", as used herein, refers to an alkenyl group with 2 or more carbon atoms having one or more double bonds in the "$C_1$-$_{18}$ alkyl group" and includes an alkadienyl group, an alkatrienyl group, and the like.

The "$C_{2-18}$ alkenyl group" is, for example, but not limited to, a vinyl group (ethenyl group), an allyl group (2-propenyl group), a 1-propenyl group, an isopropenyl group (1-methyl vinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "$C_{2-18}$ alkynyl group", as used herein, refers to an alkynyl group with 2 or more carbon atoms having one or more triple bonds in the "$C_1$-$_{18}$ alkyl group".

The "$C_{2-18}$ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "$C_{3-18}$ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "$C_{3-18}$ alicyclic group" may be replaced by -O-, -C(=O)-, -C(=O)O-, -OCO-, -CONH-, - NHCO-, -NH(C=O)O-, -S-, or -$SO_2$- (provided that adjacent divalent carbon atoms are not replaced at the same time).

The "$C_{3-18}$ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbonyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the

like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "$C_{2-18}$ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "$C_{2-18}$ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "$C_{7-18}$ aralkyl group", as used herein, refers to a group in which a substitutable portion of a "$C_{1-12}$ alkyl group" is substituted by the "$C_{2-12}$ aryl group".

The "$C_{7-18}$ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "$C_1$-$_{18}$ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "$C_1$-$_{18}$ hydrocarbyl group" may be replaced by -O-, -C(=O)-, -C(=O)O-, -OCO-, -CONH-, -NHCO-, -NH(C=O)O-, -S-, or $-SO_2-$ (provided that adjacent divalent carbon atoms are not replaced at the same time). The same applies to the "$C_1$-$_{18}$ hydrocarbyl group" and the like used in the following description of the definition of the "$C_1$-$_{18}$ hydrocarbyloxy group" and the like.

The "$C_1$-$_{18}$ hydrocarbyl group" is, for example, but not limited to, a "$C_1$-$_{18}$ alkyl group", a "$C_{2-18}$ alkenyl group", a "$C_{2-18}$ alkynyl group", a "$C_{3-18}$ alicyclic group", a "$C_{2-18}$ aryl group", a "$C_{7-18}$ aralkyl group", or the like.

The term "$C_1$-$_{18}$ hydrocarbyloxy group", as used herein, refers to a group in which an oxygen atom (-O-) is bonded to the "$C_1$-$_{18}$ hydrocarbyl group".

The "$C_1$-$_{18}$ hydrocarbyloxy group" is, for example, but not limited to, a "$C_1$-$_{18}$ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "$C_{3-18}$ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "$C_{6-18}$ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "$C_1$-$_{18}$ hydrocarbyloxy group", a divalent carbon atom at any position excluding the terminals in the "$C_1$-$_{18}$ hydrocarbyl group" is preferably replaced by -O-, - C(=O)-, and/or -C(=O)O-, the "$C_1$-$_{18}$ hydrocarbyloxy group" is more preferably a "$C_1$-$_{18}$ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "$C_1$-$_{18}$ hydrocarbylcarbonyl group", as used herein, refers to a group in which a carbonyl group (-C(=O)-) is bonded to the "$C_1$-$_{18}$ hydrocarbyl group".

The "$C_1$-$_{18}$ hydrocarbylcarbonyl group" is, for example, but not limited to, a "$C_1$-$_{18}$ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "$C_{3-18}$ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "$C_{6-18}$ aryl carbonyl group", such as a benzoyl group, a 1-naphthoyl group, or a 2-naphthoyl group; or the like.

The term "$C_1$-$_{18}$ hydrocarbylcarbonyloxy group", as used herein, refers to a group in which an oxygen atom (-O-) is bonded to the "$C_1$-$_{18}$ hydrocarbylcarbonyl group".

The "$C_1$-$_{18}$ hydrocarbylcarbonyloxy group" is, for example, but not limited to, a "$C_1$-$_{18}$ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "$C_{3-18}$ alicyclic carbonyloxy group", such as a cyclopropyl carbonyloxy group, a cyclobutyl carbonyloxy group, a cyclopentyl carbonyloxy group, or a cyclohexyl carbonyloxy group; a "$C_{6-18}$ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "$C_1$-$_{18}$ hydrocarbyloxycarbonyl group", as used herein, refers to a group in which a carbonyl group (-C(=O)-) is bonded to a "$C_1$-$_{18}$ hydrocarbyloxy group".

The "$C_1$-$_{18}$ hydrocarbyloxycarbonyl group" is, for example, but not limited to, a "$C_1$-$_{18}$ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "$C_{3-18}$ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "$C_{6-18}$ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "$C_1$-$_{18}$ hydrocarbyloxycarbonyloxy group", as used herein, refers to a group in which an oxygen atom (-O-) is bonded to the "$C_1$-$_{18}$ hydrocarbyloxycarbonyl group".

The "$C_1$-$_{18}$ hydrocarbyloxycarbonyloxy group" is, for example, but not limited to, a "$C_1$-$_{18}$ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "$C_{3-18}$ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "$C_{6-18}$ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "$C_1$-$_{18}$ hydrocarbylamino group", as used herein, refers to a group in which one "$C_1$-$_{18}$ hydrocarbyl group" is bonded to an amino group.

The "$C_1$-$_{18}$ hydrocarbylamino group" is, for example, but not limited to, a "$C_1$-$_{18}$ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "$C_{3-18}$ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "$C_{6-18}$ arylamino group", such as a phenylamino group, a 1-naphthylamino group, or a 2-naphthylamino group; or the like.

The term "di-$C_{1-18}$ hydrocarbylamino group", as used herein, refers to a group in which two identical or different "$C_1$-$_{18}$ hydrocarbyl groups" are bonded to an amino group.

The "di-$C_{1-18}$ hydrocarbylamino group" is, for example, but not limited to, a "di-$C_1$-$_{18}$ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-$C_{3-18}$ alicyclic amino group", such as a dicyclopropylamino group, a dicyclobutylamino group, a dicyclopentylamino group, or a dicyclohexylamino group; a "di-$C_{6-18}$ arylamino group", such as a diphenylamino group or a phenylnaphthylamino group; an "N-$C_1$-$_{18}$ alkyl-N-$C_{3-18}$ cycloalkylamino group", such as an N-methylcyclopentaneamino group or an N-methylcyclohexylamino group; an "N-$C_1$-$_{18}$ alkyl-N-$C_{6-18}$ arylamino group", such as an N-methyl-2-phenylethylamino group or an N-ethyl-N-(4-methylphenyl)amino group; or the like.

The term "$C_1$-$_{18}$ hydrocarbylaminocarbonyl group", as used herein, refers to a group in which a carbonyl group

(-C(=O)-) is bonded to the "$C_1$-$_{18}$ hydrocarbylamino group".

The "$C_1$-$_{18}$ hydrocarbylaminocarbonyl group" is, for example, but not limited to, a "$C_1$-$_{18}$ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "$C_{3-18}$ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "$C_{6-18}$ arylaminocarbonyl group", such as a phenylaminocarbonyl group, a 1-naphthylaminocarbonyl group, or a 2-naphthylaminocarbonyl group.

The term "di-$C_{1-18}$ hydrocarbylaminocarbonyl group", as used herein, refers to a group in which a carbonyl group (-C(=O)-) is bonded to a "di-$C_{1-18}$ hydrocarbylamino group".

The "di-$C_{1-18}$ hydrocarbylaminocarbonyl group" is, for example, but not limited to, a "di-$C_{1-18}$ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-$C_{3-18}$ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-$C_{6-18}$ arylaminocarbonyl group", such as a diphenylaminocarbonyl group or a phenylnaphthylaminocarbonyl group; or the like.

The term "$C_1$-$_{18}$ hydrocarbylcarbonylamino group", as used herein, refers to a group in which an amino group is bonded to the "$C_1$-$_{18}$ hydrocarbylcarbonyl group".

The "$C_1$-$_{18}$ hydrocarbylcarbonylamino group" is, for example, but not limited to, a "$C_1$-$_{18}$ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "$C_{3-18}$ alicyclic carbonylamino group", such as a cyclopropyl carbonylamino group, a cyclobutyl carbonylamino group, a cyclopentyl carbonylamino group, or a cyclohexyl carbonylamino group; a "$C_{6-18}$ aryl carbonylamino group", such as a phenyl carbonylamino group, a naphthyl carbonylamino group, an acenaphthyl carbonylamino group, a phenanthrenyl carbonylamino group, or an anthracenyl carbonylamino group; or the like.

The term "$C_1$-$_{18}$ hydrocarbylaminocarbonyloxy group", as used herein, refers to a group in which an oxygen atom (-O-) is bonded to the "$C_1$-$_{18}$ hydrocarbylaminocarbonyl group".

The "$C_1$-$_{18}$ hydrocarbylaminocarbonyloxy group" is, for example, but not limited to, a "$C_1$-$_{18}$ alkylaminocarbonyloxy group", such as a methylaminocarbonyloxy group, an ethylaminocarbonyloxy group, or a n-propylaminocarbonyloxy group; a "$C_{3-18}$ alicyclic aminocarbonyloxy group", such as a cyclopropylaminocarbonyloxy group or a cyclohexylaminocarbonyloxy group; a "$C_{6-18}$ arylaminocarbonyloxy group", such as a phenylaminocarbonyloxy group or a 1-naphthylaminocarbonyloxy group; or the like.

The term "di-$C_{1-18}$ hydrocarbylaminocarbonyloxy group", as used herein, refers to a group in which an oxygen atom (-O-) is bonded to the "di-$C_{1-18}$ hydrocarbylaminocarbonyl group".

The "di-$C_{1-18}$ hydrocarbylaminocarbonyloxy group" is, for example, but not limited to, a "di-$C_{1-18}$ alkylaminocarbonyloxy group", such as a dimethylaminocarbonyloxy group, a diethylaminocarbonyloxy group, or a di-n-propylaminocarbonyloxy group; or the like.

The term "$C_1$-$_{18}$ hydrocarbylaminocarbonylamino group", as used herein, refers to a group in which the "$C_1$-$_{18}$ hydrocarbylaminocarbonyl group" is bonded to an amino group.

The "$C_1$-$_{18}$ hydrocarbylaminocarbonylamino group" is, for example, but not limited to, a "$C_1$-$_{18}$ alkylaminocarbonylamino group", such as a methylaminocarbonylamino group, an ethylaminocarbonyloxy group, or a n-propylaminocarbonylamino group; a "$C_{3-18}$ alicyclic aminocarbonylamino group", such as a cyclopropylaminocarbonylamino group or a cyclohexylaminocarbonylamino group; a "$C_{6-18}$ arylaminocarbonylamino group", such as a phenylaminocarbonylamino group or a 1-naphthylaminocarbonylamino group; or the like.

The term "di-$C_{1-18}$ hydrocarbylaminocarbonylamino group", as used herein, refers to a group in which a "di-$C_{1-18}$ hydrocarbylaminocarbonyl group" is bonded to an amino group.

The "di-$C_{1-18}$ hydrocarbylaminocarbonylamino group" is, for example, but not limited to, a "di-$C_{1-18}$ alkylami-

nocarbonylamino group", such as a dimethylaminocarbonylamino group, a diethylaminocarbonylamino group, or a di-n-propylaminocarbonylamino group; or the like.

The term "$C_1$-$_{18}$ hydrocarbyloxycarbonylamino group", as used herein, refers to a group in which the "$C_1$-$_{18}$ hydrocarbyloxycarbonyl group" is bonded to an amino group.

The "$C_1$-$_{18}$ hydrocarbyloxycarbonylamino group" is, for example, but not limited to, a "$C_1$-$_{18}$ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "$C_{3-18}$ alicyclic oxycarbonylamino group", such as a cyclopropyloxycarbonylamino group or a cyclohexyloxycarbonylamino group; a "$C_{6-18}$ aryloxycarbonylamino group", such as a phenyloxycarbonylamino group or a 1-naphthyloxycarbonylamino group; or the like.

The term "$C_1$-$_{18}$ hydrocarbylthio group", as used herein, refers to a group in which a sulfur atom (-S-) is bonded to the "$C_1$-$_{18}$ hydrocarbyl group".

The "$C_1$-$_{18}$ hydrocarbylthio group" is, for example, but not limited to, a "$C_1$-$_{18}$ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "$C_{3-18}$ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "$C_{6-18}$ arylthio group", such as a phenylthio group, a 1-naphthylthio group, a 2-naphthylthio group, an acenaphthylthio group, a phenanthrenylthio group, or an anthracenylthio group; or the like.

The term "$C_1$-$_{18}$ hydrocarbylsulfinyl group", as used herein, refers to a group in which a sulfinyl group (-S(=O)-) is bonded to the "$C_1$-$_{18}$ hydrocarbyl group".

The "$C_1$-$_{18}$ hydrocarbylsulfinyl group" is, for example, but not limited to, a "$C_1$-$_{18}$ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "$C_{3-18}$ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "$C_{6-18}$ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "$C_1$-$_{18}$ hydrocarbylsulfonyl group", as used herein, refers to a group in which a sulfonyl group ($-SO_2-$) is bonded to the "$C_1$-$_{18}$ hydrocarbyl group".

The "$C_1$-$_{18}$ hydrocarbylsulfonyl group" is, for example, but not limited to, a "$C_1$-$_{18}$ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "$C_{3-18}$ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "$C_{6-18}$ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes a hydrogen atom of a carboxy group or a hydroxy group (including a phenolic hydroxy group or the like) and is dissociated by the action of an acid.

The term "acid-dissociable group" is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

The phrase "optionally substituted", as used herein, is not particularly limited, provided that it is chemically acceptable and has the advantages of the present invention.

The "substituent" is, for example, (1) a halogen atom, (2) a hydroxy group, (3) a thiol group, (4) a nitro group, (5) a cyano group, (6) a carboxy group, (7) an amino group, (8) a sulfo group, (9) a group in which a hydrogen atom of a carboxy group or a hydroxy group is substituted by an acid-dissociable group,
or (10) a $C_{1-8}$ hydrocarbyl group, a $C_{1-8}$ hydrocarbyloxy group, a $C_{1-8}$ hydrocarbylcarbonyl group, a $C_{1-8}$ hydrocarbylcarbonyloxy group, a $C_{1-8}$ hydrocarbyloxycarbonyl group, a $C_{1-8}$ hydrocarbyloxycarbonyloxy group, a $C_{1-8}$ hydrocarbylamino group, a di-$C_{1-8}$ hydrocarbylamino group, a $C_{1-8}$ hydrocarbylaminocarbonyl group, a di-$C_{1-8}$ hydrocarbylaminocarbonyl group, a $C_{1-8}$ hydrocarbylcarbonylamino group, a $C_{1-8}$ hydrocarbylaminocarbonyloxy group, a di-$C_{1-8}$ hydrocarbylaminocarbonyloxy group, a $C_{1-8}$ hydrocarbylaminocarbonylamino group, a di-$C_{1-8}$ hydrocarbylaminocarbonylamino group, a $C_{1-8}$ hydrocarbyloxycarbonylamino group, a $C_{1-8}$ hydrocarbylthio group, a $C_{1-8}$ hydrocarbylsulfinyl group, a $C_{1-8}$ hydrocarbylsulfonyl group, in which at least part of their hydrogen atoms may be substituted by (1) to (9) and a divalent carbon atom at any position except the end(s) of the substituent may be replaced by -O-, -C(=O)-, -C(=O)O-, - OCO-, -CONH-, -NHCO-, -NH(C=O)O-, -S-, or $-SO_2-$ (provided that adjacent divalent carbon atoms are not replaced at the same time), or the like.

[1. Method for Producing Metatungstic Acid Salt Compound]

A method for producing a metatungstic acid salt compound according to one embodiment of the present invention is a method for producing a metatungstic acid salt compound represented by the following General Formula (I-1) or (I-2) (hereinafter also referred to as a "metatungstic acid salt compound (I-1)" or a "metatungstic acid salt compound (I-2)"), the method including a step of performing salt exchange in an acidic solution having a pH of less than 3.

$$(A^+)_5 \bullet [H_3W_{12}O_{40}]^{5-} \qquad (I\text{-}1)$$

$$(A'^{2+})_5 \bullet ([H_3W_{12}O_{40}]^{5-})_2 \qquad (I\text{-}2)$$

[in Formulae (I-1) and (I-2),

each $A^+$ independently represents an onium cation; and
each $A'^{2+}$ independently represents an onium dication.]

Note that the description "$[H_3W_{12}O_{40}]^{5-}$" in the present description includes both a case where protons are contained inside the Keggin shell and a case where protons are contained outside the Keggin shell ($(H^+)\cdot[H_2W_{12}O_{40}]^{6-}$).

[1-1. Salt Exchange Step]

A metatungstic acid salt compound represented by General Formula (I-1) or (I-2) can be selectively produced by performing salt exchange between a metatungstic acid salt represented by the following General Formula (II) (hereinafter also referred to as a "metatungstic acid salt (II)") and an onium salt represented by the following General Formula (III-1) or (III-2) (hereinafter also referred to as an "onium salt (III-1)" or an "onium salt (III-2)") in an acidic solution having a pH of less than 3.

$$(B^+)_6 \bullet [H_2W_{12}O_{40}]^{6-} \qquad (II)$$

[in Formula (II),

each $B^+$ independently represents $H^+$, a metal ion, or an ammonium ion.]

$$A^+ \bullet X^- \qquad (III\text{-}1)$$

$$A'^{2+} \bullet (X^-)_2 \qquad (III\text{-}2)$$

[in Formulae (III-1) and (III-2),
$A^+$ represents an onium cation;
$A'^{2+}$ represents an onium dication; and
$X^-$ represents a monovalent counter anion.]

[1-1-1. Metatungstic Acid Salt (II)]

The metatungstic acid salt (II) used in this embodiment is not particularly limited, and a known and commonly used one can be used.

$$(B^+)_6 \bullet [H_2W_{12}O_{40}]^{6-} \qquad (II)$$

Metatungstic acid is a kind of isopolyoxotungstic acid, which is an anionic metal oxide cluster, and is represented as $[H_2W_{12}O_{40}]^{6-}$.

In General Formula (II), each $B^+$ independently represents $H^+$, a metal ion, or an ammonium ion. One or more kinds of these counter cations may be contained in the metatungstic acid salt (II).

From the viewpoint of promoting the salt exchange reaction, $B^+$ is preferably $H^+$, $Li^+$, $Na^+$, $K^+$, $Rb^+$, $Cs^+$, $Be^{2+}$, $Mg^{2+}$, $Ca^{2+}$, $Sr^{2+}$, $Ba^{2+}$, $Co^{3+}$, $Bi^{3+}$, $Zr^{4+}$, $Hf^{4+}$, $Bi^{5+}$, $NH^{4+}$, or a combination thereof, more preferably $H^+$, $Na^+$, $K^+$, $Mg^{2+}$, $Ca^{2+}$, $NH^{4+}$, or a combination thereof, and further preferably $H^+$, $Na^+$, $K^+$, $NH^{4+}$, or a combination thereof.

Examples of commercially available metatungstic acid salts (II) include ammonium metatungstate hydrate manufactured by Merck KGaA; and ammonium metatungstate manufactured by Nippon Inorganic Colour & Chemical Co.,

Ltd.

[1-1-2. Onium Salt (III-1) or (III-2)]

The onium salt (III-1) or (III-2) used in this embodiment is not particularly limited, and a known and commonly used one can be used. $A^+$, which is the cation moiety of the onium salt (III-1), represents an onium cation, and $X^-$, which is the anion moiety of the onium salt (III-1), represents a monovalent counter anion. $A'^{2+}$, which is the cation moiety of the onium salt (III-2), represents an onium dication, and $X^-$, which is the anion moiety of the onium salt (III-2), represents a monovalent counter anion.

$$A^+ \bullet X^- \qquad (III\text{-}1)$$

$$A'^{2+} \bullet (X^-)_2 \qquad (III\text{-}2)$$

[1-1-2-1. Cation Moiety of Onium Salt (III-1) or (III-2)]

From the viewpoint of improving actinic radiation sensitivity and/or radiation sensitivity, $A^+$ or $A'^{2+}$ is, among onium cations or onium dications, preferably an onium cation or onium dication having at least one unsaturated bond, which may have a substituent. Examples of the unsaturated bond include a carbon-carbon double bond and a carbon-carbon triple bond. Among these, a carbon-carbon double bond is more preferable, and a benzene ring is further preferable. Examples of the onium cation include an organic sulfonium cation, an organic iodonium cation, and an organic quaternary ammonium cation. Examples of the onium dication include an organic sulfonium dication, an organic iodonium dication, and an organic ammonium dication.

[1-1-2-1-1. Organic Sulfonium Cation or Organic Sulfonium di-Cation]

The organic sulfonium cation is not particularly limited and may be a known and commonly used organic sulfonium cation. The organic sulfonium cation is, for example, an organic sulfonium cation represented by the formula (IV).

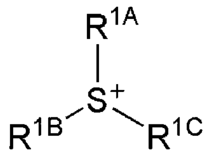 (IV)

In the formula (IV), $R^{1A}$, $R^{1B}$, and $R^{1C}$ each independently denote a $C_{1-18}$ hydrocarbyl group, a 3- to 18-membered non-aromatic heterocyclic group, or a 5- to 18-membered aromatic heterocyclic group,

a divalent carbon atom at any position except the end(s) of $R^{1A}$, $R^{1B}$, and $R^{1C}$ may be replaced by -O-, -C(=O)-, -C(=O)O-, -OCO-, -CONH-, -NHCO-, -NH(C=O)O-, -S-, or $-SO_2-$ (provided that adjacent divalent carbon atoms are not replaced at the same time),
a hydrogen atom in $R^{1A}$, $R^{1B}$, and $R^{1C}$ is, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, or a $C_{1-18}$ hydrocarbylthio group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, -NH(C=O)O-, -S-, or $-SO_2-$ (provided that adjacent divalent carbon atoms are not replaced at the same time); and
any two of $R^{1A}$, $R^{1B}$, and $R^{1C}$ may be linked to each other directly by a single bond or via a divalent linking group -O-, -S-, -C(=O)-, -S(=O)-, $-S(=O)_2-$, -C(=O)O-, or a $C_{1-3}$ alkylene group to form a ring together with a sulfur atom in the (IV).

$R^{1A}$, $R^{1B}$, and $R^{1C}$ in the formula (IV) preferably each independently denote an optionally substituted $C_{6-18}$ aryl group. A substituent for the $C_{6-18}$ aryl group is preferably a halogen atom, a haloalkyl group, a hydroxy group, a nitro group, a cyano group, or an optionally substituted $C_{1-12}$ hydrocarbylthio group, more preferably a halogen atom, a $C_{1-4}$ haloalkyl group, or a nitro group.

The organic sulfonium di-cation is not particularly limited and may be a known and commonly used organic sulfonium di-cation. The organic sulfonium di-cation is, for example, an organic sulfonium di-cation represented by the formula (V).

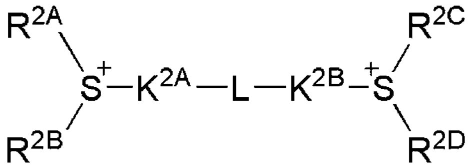
(V)

In Formula (V), $R^{2A}$, $R^{2B}$, $R^{2C}$, and $R^{2D}$ each independently represent a $C_{1-18}$ hydrocarbyl group that may have a substituent, a 3- to 18-membered non-aromatic heterocyclic group, or a 5- to 18-membered aromatic heterocyclic group, and

any divalent carbon atom at any position other than the terminals of $R^{2A}$, $R^{2B}$, $R^{2C}$, and $R^{2D}$ may be replaced with -O-, -C(=O)-, -C(=O)O-, -OCO-, -CONH-, -NHCO-, - NH(C=O)O-, -S-, or $-SO_2-$ (provided that adjacent divalent carbon atoms are not both replaced);

$K^{2A}$, $K^{2B}$, and L each independently represent a $C_{1-18}$ hydrocarbylene group that may have a substituent, and any divalent carbon atom at any position of $K^{2A}$, $K^{2B}$, and L may be replaced with - O-, -C(=O)-, -C(=O)O-, -OCO-, -CONH-, -NHCO-, -S-, or $-SO_2-$ (provided that adjacent divalent carbon atoms are not both replaced);

the hydrogen atoms contained in $R^{2A}$ , $R^{2B}$, $R^{2C}$, $R^{2D}$, $K^{2A}$, $K^{2B}$, and L are each, for example, (a) a halogen atom, (b) a haloalkyl group, (c) a hydroxyl group, (d) a thiol group, (e) a nitro group, (f) a cyano group, (g) a carboxyl group, (h) an amino group, (i) a sulfo group, or (j) a $C_{1-18}$ hydrocarbyl group, a $C_{1-18}$ hydrocarbyloxy group, a $C_{1-18}$ hydrocarbylcarbonyl group, a $C_{1-18}$ hydrocarbylcarbonyloxy group, a $C_{1-18}$ hydrocarbyloxycarbonyl group, a $C_{1-18}$ hydrocarbyloxycarbonyloxy group, or a $C_{1-18}$ hydrocarbylthio group, in each of which at least a part of the hydrogen atoms may be substituted with any of the (a) to (i), and any divalent carbon atom at any position other than the terminals of the above substituents may be replaced with -O-, -C(=O)-, -C(=O)O-, -OCO-, -CONH-, -NHCO-, -NH(C=O)O-, -S-, or $-SO_2-$ (provided that adjacent divalent carbon atoms are not both replaced); and

any two of $R^{2A}$, $R^{2B}$, and $K^{2A}$, and/or any two of $R^{2C}$, $R^{2D}$, and $K^{2B}$ may be directly bonded to each other with a single bond, or bonded with a divalent linking group of -O-, - S-, -C(=O)-, -S(=O)-, $-S(=O)_2-$, -C(=O)O-, or a $C_{1-3}$ alkylene group, to form a ring together with the sulfur atom in Formula (V).

Specific examples of the organic sulfonium cation or the organic sulfonium di-cation include a dibutyl(pentyl) sulfonium cation, a triethylsulfonium cation, (2-carboxyethyl)dimethylsulfonium cation, a trimethylsulfonium cation, a dimethylphenacylsulfonium cation, a 1-(4-hydroxynaphthalen-1-yl)hexahydrothiopyrylium cation, a dimethylphenylsulfonium cation, a triphenylsulfonium cation, a tris(4-methylphenyl)sulfonium cation, a 4-methoxyphenyldiphenylsulfonium cation, a 4-iodophenyldiphenylsulfonium cation, a tris(4-fluorophenyl)sulfonium cation, a 1-phenylhexahydrothiopyrylium cation, an (ethane-1,2-diylbisoxy)bis(4,1-phenylene)bis(diphenylsulfonium) dication, a (thiodi-4,1-phenylene)bis(diphenylsulfonium) dication, a di(naphthalen-1-yl)(phenyl)sulfonium cation, a phenyl bis(2-(trifluoromethyl)phenyl)sulfonium cation, a mesityl bis(2-(trifluoromethyl)phenyl)sulfonium cation, a bis(3,5-difluorophenyl)(phenyl)sulfonium cation, a tris(3,5-difluorophenyl)sulfonium cation, a (4-(dodecanoyloxy-3,5-dimethylphenyl))diphenylsulfonium cation, a diphenyl(3-(trifluoromethoxy)phenyl)sulfonium cation, a (4-(1-adamantylcarbonyloxy)phenyl)diphenylsulfonium cation, a (4-phenylthiophenyl)diphenylsulfonium cation, a 5-phenyl-5H-thianthren-5-ium cation, a 5-(2,5-dimethylphenyl)thianthren-5-ium cation, a 5-phenyl-5H-dibenzo[b,d]thiophen-5-ium cation, a 5-(3-(trifluoromethyl)phenyl)-5H-dibenzo[b,d]thiophen-5-ium cation, a 1-(4-(t-butyl)phenyl)-1H-benzo[b]thiophen-1-ium cation, a methyldiphenylsulfonium cation, a (2-bromoethyl)diphenylsulfonium cation, a (3-chloropropyl)diphenylsulfonium cation, a benzyl(4-hydroxyphenyl)methylsulfonium cation, a (4-hydroxyphenyl)methyl(2-methylbenzyl)sulfonium cation, a 4-hydroxyphenyldimethylsulfonium cation, a diphenyl(methyl)sulfonium cation, a diphenyl(4-(phenylthio)phenyl)sulfonium cation, a (2-bromoethyl)diphenylsulfonium cation, a dimesityl(trifluoromethyl)sulfonium cation, and a tri-p-tolylsulfonium cation.

[1-1-2-1-2. Organic Iodonium Cation]

The organic iodonium cation is not particularly limited and may be a known and commonly used organic iodonium cation. The organic iodonium cation is, for example, an organic iodonium cation represented by the formula (VI).

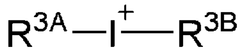
(VI)

In the formula (VI), $R^{3A}$ and $R^{3B}$ each independently denote a $C_1$-$_{18}$ hydrocarbyl group, a 3- to 18-membered non-aromatic heterocyclic group, or a 5- to 18-membered aromatic heterocyclic group

a divalent carbon atom at any position except the end(s) of $R^{3A}$ and $R^{3B}$ may be replaced by -O-, -C(=O)-, -C(=O)O-, -OCO-, -CONH-, -NHCO-, -NH(C=O)O-, -S-, or - $SO_2$- (provided that adjacent divalent carbon atoms are not replaced at the same time);

a hydrogen atom in $R^{3A}$ and $R^{3B}$ is, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a $C_1$-$_{18}$ hydrocarbyl group, a $C_1$-$_{18}$ hydrocarbyloxy group, a $C_1$-$_{18}$ hydrocarbylcarbonyl group, a $C_1$-$_{18}$ hydrocarbylcarbonyloxy group, a $C_1$-$_{18}$ hydrocarbyloxycarbonyl group, or a $C_1$-$_{18}$ hydrocarbyloxycarbonyloxy group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, - NHCO-, -NH(C=O)O-, -S-, or -$SO_2$- (provided that adjacent divalent carbon atoms are not replaced at the same time); and

furthermore, $R^{3A}$ and $R^{3B}$ may be linked to each other directly by a single bond or via a divalent linking group -O-, -S-, -C(=O)-, -S(=O)-, -S(=O)$_2$-, -C(=O)O-, or a $C_{1-3}$ alkylene group to form a ring together with the iodine atom in the formula (VI).

$R^{3A}$ and $R^{3B}$ in the formula (VI) preferably each independently denote an optionally substituted $C_{6-18}$ aryl group. A substituent for the $C_{6-18}$ aryl group is preferably a halogen atom, a haloalkyl group, a hydroxy group, a nitro group, a cyano group, an optionally substituted $C_{1-12}$ hydrocarbyl group, $C_{1-12}$ hydrocarbyloxy group, $C_{1-12}$ hydrocarbylcarbonyl group, $C_{1-12}$ hydrocarbylcarbonyloxy group, $C_{1-12}$ hydrocarbyloxycarbonyl group, or $C_{1-12}$ hydrocarbyloxycarbonyloxy group, preferably a halogen atom, a $C_{1-4}$ haloalkyl group, a nitro group, a $C_{1-8}$ hydrocarbyl group, or a $C_{1-8}$ hydrocarbyloxy group.

Specific examples of the iodonium cations include an ethynyl(phenyl)iodonium cation, a bis(pyridine)iodonium cation, a bis(2,4,6-trimethylpyridine)iodonium cation, a diphenyliodonium cation, a bis(4-(t-butyl)phenyl)iodonium cation, a (2-carboxyphenyl)(phenyl)iodonium cation, a (4-nitrophenyl)(phenyl)iodonium cation, a (3-(trifluoromethyl)phenyl)(2,4,6-trimethylphenyl)iodonium cation, a bis(4-fluorophenyl)iodonium cation, a (4-(bromomethyl)phenyl)(2,4,6-trimethoxyphenyl)iodonium cation, a 4-biphenylyl(2,4,6-trimethoxyphenyl)iodonium cation, a bis(2,4,6-trimethylphenyl)iodonium cation, a 4-isopropyl-4'-methyldiphenyliodonium cation, a (4-(trifluoromethyl)phenyl)(2,4,6-trimethylphenyl)iodonium cation, a ((4-trifluoromethyl)phenyl)(2,4,6-trimethoxyphenyl)iodonium cation, a (5-fluoro-2-nitrophenyl)(2,4,6-trimethoxyphenyl)iodonium cation, a (3-bromophenyl)(mesityl)iodonium cation, a bis(4-bromophenyl)iodonium cation, a (3,5-dichlorophenyl)(2,4,6-trimethoxyphenyl)iodonium cation, a (4-methylphenyl)(2,4,6-trimethylphenyl)iodonium cation, a (3-methylphenyl)(2,4,6-trimethylphenyl)iodonium cation, a (2-methylphenyl)(2,4,6-trimethylphenyl)iodonium cation, a phenyl(2,4,6-trimethoxyphenyl)iodonium cation, and the like.

[1-1-2-1-3. Organic Quaternary Ammonium Cation]

The organic quaternary ammonium cation is not particularly limited and may be a known and commonly used organic quaternary ammonium cation. The organic quaternary ammonium cation is, for example, an organic quaternary ammonium cation represented by the formula (VII).

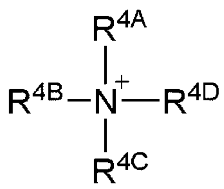

(VII)

In the formula (VII), $R^{4A}$, $R^{4B}$, $R^{4C}$, and $R^{4D}$ each independently denote a $C_{1-18}$ alkyl group, a $C_{1-18}$ alkynyl group, a $C_{1-18}$ alicyclic group, a 3- to 18-membered non-aromatic heterocyclic group, or a 5- to 18-membered aromatic heterocyclic group,

a divalent carbon atom at any position except the end(s) of $R^{4A}$, $R^{4B}$, $R^{4C}$, and $R^{4D}$ may be replaced by -O-, -C(=O)-, -C(=O)O-, -OCO-, -CONH-, -NHCO-, -NH(C=O)O-, - S-, or -$SO_2$- (provided that adjacent divalent carbon atoms are not replaced at the same time);

a hydrogen atom in $R^{4A}$, $R^{4B}$, $R^{4C}$, and $R^{4D}$ is, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, or (j) a $C_1$-$_{18}$ hydrocarbyl group, a $C_1$-$_{18}$ hydrocarbyloxy group, a $C_1$-$_{18}$ hydrocarbylcarbonyl group, a $C_1$-$_{18}$ hydro-

carbylcarbonyloxy group, a $C_{1-18}$ hydrocarbyloxycarbonyl group, a $C_{1-18}$ hydrocarbyloxycarbonyloxy group, or a $C_{1-18}$ hydrocarbylthio group, in which at least part of a hydrogen atom may be substituted by (a) to (i), and a divalent carbon atom at any position except the ends of these substituents may be replaced by -O-, -C(=O)-, -C(=O)O-, -OCO-, -CONH-, -NHCO-, -NH(C=O)O-, -S-, or $-SO_2-$ (provided that adjacent divalent carbon atoms are not replaced at the same time); and

furthermore, any two of $R^{4A}$, $R^{4B}$, $R^{4C}$, and $R^{4D}$ may be linked to each other directly by a single bond or via a divalent linking group -O-, -S-, -C(=O)-, -S(=O)-, $-S(=O)_2-$, - C(=O)O-, or a $C_{1-3}$ alkylene group to form a ring together with the nitrogen atom in the formula (VII).

$R^{4A}$, $R^{4B}$, $R^{4C}$, and $R^{4D}$ in the formula (VII) preferably each independently denote an optionally substituted $C_{1-18}$ hydrocarbyl group, more preferably a $C_{1-12}$ alkyl group, a $C_{1-12}$ alkynyl group, or a $C_{1-12}$ alicyclic group.

Specific examples of the organic quaternary ammonium cation include a tetramethylammonium cation, a tetraethylammonium cation, a tetrapropylammonium cation, a tetrabutylammonium cation, a tetraheptylammonium cation, a trimethylethylammonium cation, a dimethyldiethylammonium cation, a dimethylethylpropylammonium cation, a methylethylpropylbutylammonium cation, a trimethylphenylammonium cation, a triethylhexylammonium cation, a triethylcyclohexylammonium cation, and a dodecyltrimethylammonium cation.

[1-1-2-2. Anion Moiety of Onium Salt (III-1) or (III-2)]

$X^-$, which is the anion moiety of the onium salt (III-1) or (III-2), represents a monovalent counter anion.

$$A^+ \bullet X^- \qquad \text{(III-1)}$$

$$A'^{2+} \bullet (X^-)_2 \qquad \text{(III-2)}$$

$X^-$ is not particularly limited, and examples thereof include inorganic anions and organic anions.

Examples of the inorganic anion or the organic anion include halide ions such as $F^-$, $Cl^-$, $Br^-$, and $I^-$; hydroxide ions; sulfonate ions such as $HSO_4^-$, $CF_3SO_3^-$, $C_2F_5SO_3^-$, $C_3F_7SO_3^-$, $C_4F_9SO_3^-$, p-$CH_3(C_6H_4)SO_3^-$, and $ClSO_3^-$; perchlorate ions; nitrate ions; borate ions such as $(C_6F_5)_4B^-$ and $BF_4^-$; fluorophosphate ions such as $PF_6^-$, $(PF_5OH)^-$, and $((CF_3CF_2)_3PF_3)^-$; imide ions such as $(CF_3SO_2)_2N^-$ and $(FSO_2)_2N^-$; antimonate ions such as $SbF_6^-$; arsenate ions such as $AsF_6^-$; $AlCl_4^-$; and $BiF_6^-$.

From the perspective of promoting the salt exchange reaction, $X^-$ is preferably $Br^-$, $Cl^-$, $I^-$, $Br^{3-}$, $OH^-$, $HSO_4^-$, $CF_3SO_3^-$, $C_2F_5SO_3^-$, $C_3F_7SO_3^-$, $C_4F_9SO_3^-$, p-$CH_3(C_6H_4)SO_3^-$, $ClSO_3^-$, $ClO_4^-$, $NO_f$, $(C_6F_5)_4B^-$, $BF_4^-$, $PF_6^-$, $SbF_6^-$, $AlCl_4^-$, $BiF_6^-$, $AsF_6^-$, $(CF_3SO_2)_2N^-$, or $(FSO_2)_2N^-$, more preferably $Br^-$, $Cl^-$, $I^-$, $OH^-$, $CF_3SO_3^-$, $C_4F_9SO_3^-$, $BF_4^-$, $PF_6^-$, $SbF_6^-$, $(CF_3SO_2)_2N^-$, or $(FSO_2)_2N^-$,still more preferably $Br^-$, $Cl^-$, $CF_3SO_3^-$, $BF_4^-$, $PF_6^-$, $SbF_6^-$, $(CF_3SO_2)_2N^-$, or $(FSO_2)_2N^-$.

[1-1-2-3. Specific Examples of Onium Salt (III-1) or (III-2)]

Examples of commercially available onium salts (III-1) or (III-2) include the following compounds manufactured by FUJIFILM Wako Pure Chemical Corporation: diphenyl-4-methylphenylsulfonium trifluoromethanesulfonate), diphenyl-2,4,6-trimethylphenylsulfonium p-toluenesulfonate, diphenyl(4-methoxyphenyl)sulfonium trifluoromethanesulfonate, 4-methylphenyldiphenylsulfonium nonafluorobutanesulfonate, tris(4-methylphenyl)sulfonium nonafluorobutanesulfonate, tributylmethylammonium bis(trifluoromethanesulfonyl)imide, methyltrioctylammonium bis(trifluoromethanesulfonyl)imide, tetrabutylammonium bromide, tetrabutylammonium hexafluorophosphate, and N-ethyl-N-(2-methoxyethyl)-N,N-dimethylammonium bis(trifluoromethanesulfonyl)imide; and the following compounds manufactured by Tokyo Chemical Industry Co., Ltd.: benzyl(4-hydroxyphenyl)methylsulfonium hexafluoroantimonate, diphenyl(methyl) sulfonium tetrafluoroborate, trimethylsulfonium bromide, 4-hydroxyphenyldimethylsulfonium methylsulfate, (2-bromoethyl)diphenylsulfonium trifluoromethanesulfonate, triphenylsulfonium bromide, trimethylsulfonium hydroxide, triphenylsulfonium nonafluoro-1-butanesulfonate, triphenylsulfonium hexafluorophosphate, triphenylsulfonium hexafluoroantimonate, diphenyl(4-(phenylthio)phenyl)sulfonium hexafluoroantimonate, diphenyl(4-(phenylthio)phenyl)sulfonium hexafluorophosphate, (3-chloropropyl)diphenylsulfonium tetrafluoroborate, (2-carboxyethyl)dimethylsulfonium chloride, (2-carboxyethyl)dimethylsulfonium bromide, tri-p-tolylsulfonium trifluoromethanesulfonate, (difluoromethyl)bis(2,5-dimethylphenyl)sulfonium tetrafluoroborate, (4-hydroxyphenyl)methyl(2-methylbenzyl)sulfonium hexafluoroantimonate, tributylsulfonium iodide, (thiodi-4,1-phenylene)bis(diphenylsulfonium) bis(hexafluorophosphate), 4-isopropyl-4'-methyldiphenyliodonium tetrakis(pentafluorophenyl)borate, (4-(bromomethyl)phenyl)(2,4,6-trimethoxyphenyl)iodonium p-toluenesulfonate, 4-biphenylyl(2,4,6-trimethoxyphenyl)iodonium trifluoromethanesulfonate, bis(4-t-butylphenyl)iodonium hexafluorophosphate, bis(2,4,6-trimethylphenyl)iodonium trifluoromethanesulfonate, bis(2,4,6-trimethylpyridine)iodonium hexafluorophosphate, bis(4-fluorophenyl)iodonium trifluoromethanesulfonate, ethynyl(phenyl)iodonium tetrafluoroborate, (4-(trifluoromethyl)phenyl)(2,4,6-trimethoxyphenyl)iodonium p-toluenesulfonate, (4-(trifluoromethyl)phenyl)(2,4,6-trimethylphenyl)iodonium trifluoromethanesulfonate, diphenyliodonium chloride, diphenyliodonium bromide, diphenyliodonium hexafluorophosphate, diphenyliodonium trifluoromethanesulfonate, benzyltrimethylammonium chloride, benzyldimethyltetradecylammonium chloride, benzyldodecyldimethylammonium chloride, tetrabutylammonium chloride, tetraamylammonium chloride, benzoylcholine chloride, benzyltributylammonium chloride, tetrabutylammonium bromide, tetrahexylammonium bromide, tetraheptylammonium bromide, tetra-n-octylammonium bromide, 1-butyl-1-methylpiperidinium bromide, 1-methyl-1-propylpiperidinium bromide, benzoylcholine bromide, benzyltriethylammonium bromide, benzyltributylammonium bromide, trimethylphenylammonium bromide, 3-(trifluoromethyl)phenyltrimethylammonium bromide, 4-ethyl-4-methylmorpholinium bromide, 3-(trifluoromethyl)phenyltrimethylammonium iodide, acetylcholine iodide, acetylthiocholine iodide, benzoylcholine iodide, benzoylthiocholine iodide, tetrabutylammonium hydroxide, tetrahexylammonium hydroxide, benzyltrimethylammonium hydroxide, benzyltriethylammonium hydroxide, 3-(trifluoromethyl)phenyltrimethylammonium hydroxide, tetrabutylammonium tetrafluoroborate, tetrabutylammonium hexafluorophosphate, amyltriethylammonium bis(trifluoromethanesulfonyl)imide, 1-butyl-1-methylpyrrolidinium bis(trifluoromethanesulfonyl)imide, 1-butyl-1-methylpiperidinium bis(trifluoromethanesulfonyl)imide, methyltri-n-octylammonium hydrogensulfate, diethyl(methyl)propylammonium bis(fluorosulfonyl)imide, and 1-ethyl-1-methylpyrrolidinium tetrafluoroborate.

[1-1-3. Acidic Solution]

A metatungstic acid salt compound represented by General Formula (I-1) or (I-2) can be selectively produced by performing salt exchange between the metatungstic acid salt (II) and the onium salt (III-1) or (III-2) in an acidic solution.

The pH of the acidic solution is preferably less than 3, more preferably 2.7 or less, further preferably 2.6 or less, particularly preferably 2.5 or less, and may be about 3 or less.

The type of acid contained in the acidic solution is not particularly limited, and examples thereof include inorganic acids such as hydrochloric acid, sulfuric acid, nitric acid, sulfamic acid, and phosphoric acid; organic acids having a carboxyl group, such as tartaric acid, oxalic acid, formic acid, acetic acid, propionic acid, malic acid, lactic acid, citric acid, succinic acid, maleic acid, fumaric acid, glycolic acid, trifluoroacetic acid, and trichloroacetic acid; and organic acids having a sulfo group, such as methanesulfonic acid, ethanesulfonic acid, sulfosuccinic acid, meta-toluenesulfonic acid, and para-toluenesulfonic acid. The acid may be used alone or in combination of two or more kinds.

Depending on the solvent used, the acid is preferably hydrochloric acid, sulfuric acid, nitric acid, phosphoric acid, formic acid, oxalic acid, trichloroacetic acid, methanesulfonic acid, or meta- or para-toluenesulfonic acid, and more preferably hydrochloric acid, sulfuric acid, nitric acid, phosphoric acid, or formic acid.

As a reaction pathway and mechanism by which a metatungstic acid salt compound (I-1) or (I-2) can be selectively produced by performing a salt exchange reaction in an acidic solution, presumably, it is considered that the metatungstic acid salt (II) is present in the form of $[H_3W_{12}O_{40}]^{5-}$ as a result of being converted thereto in an acidic solution having a pH of less than 3.

[1-2. Other Steps]

The method for producing a metatungstic acid salt compound according to this embodiment may include, as other steps, general operations performed by a person skilled in the art for isolating and purifying a target compound after the salt exchange reaction. The other steps are not particularly limited, and examples thereof include steps such as quenching the reaction, adding a poor solvent to salt out the compound, filtering, separating by liquid-liquid extraction, and purifying by chromatography, recrystallization, or the like.

[1-3. Number of Onium Cations or Onium Dications Contained in Metatungstic Acid Salt Compound (I-1) or (I-2)]

A metatungstic acid salt compound (I-1) or (I-2) containing 5 onium cations or onium dications can be selectively produced by the method for producing a metatungstic acid salt compound according to this embodiment.

The number of onium cations contained in the metatungstic acid salt compound can be determined, for example, by XRF analysis or XPS analysis.

When the onium cations contained in the metatungstic acid salt compound are organic sulfonium cations or organic sulfonium dications, the molar ratio between S and W in the metatungstic acid compound can be determined, for example, by XRF analysis, using SQX analysis or the like based on a calibration curve method or a fundamental parameter (FP) method. Alternatively, the molar ratio between S and W in the metatungstic acid salt compound can be determined by XPS analysis, on the basis of the peak areas of S2p and W4f, by calculating the atomic % of each element.

When the onium cations contained in the metatungstic acid salt compound are organic iodonium cations, the molar ratio between I and W in the metatungstic acid compound can be determined, for example, by XRF analysis, using

SQX analysis or the like based on a calibration curve method or a fundamental parameter (FP) method. Alternatively, the molar ratio between I and W in the metatungstic acid salt compound can be determined by XPS analysis, on the basis of the peak areas of I3d and W4f, by calculating the atomic % of each element.

When the onium cations contained in the metatungstic acid salt compound are organic ammonium cations, the molar ratio between N and W in the metatungstic acid compound can be determined, for example, by XRF analysis, using SQX analysis or the like based on a calibration curve method or a fundamental parameter (FP) method. Alternatively, the molar ratio between N and W in the metatungstic acid salt compound can be determined by XPS analysis, on the basis of the peak areas of N1s and W4f, by calculating the atomic % of each element.

[2. Metatungstic Acid Salt Compound for Resists]

It has been difficult to selectively produce a metatungstic acid salt compound represented by General Formula (I-1) or (I-2) containing 5 onium cations or onium dications with respect to metatungstic acid ($[H_2W_{10}O_{40}]^{6-}$), which is a hexavalent anion. That is, when produced by usual methods, a mixture of metatungstic acid salt compounds containing 4 to 6 onium cations or onium dications is obtained, and it has been impossible to selectively obtain a metatungstic acid salt compound containing 5 onium cations or onium dications.

In contrast, according to the method for producing a metatungstic acid salt compound according to this embodiment, it becomes possible to selectively produce a metatungstic acid salt compound (I-1) or (I-2) containing 5 onium cations or onium dications. Since the metatungstic acid salt compound (I-1) or (I-2) has improved actinic radiation sensitivity and/or radiation sensitivity due to the onium cations or onium dications, and also has uniformity, it can be suitably used as a metatungstic acid salt compound for resists.

[3. Resist Material]

A resist material according to this embodiment contains the metatungstic acid salt compound (I-1) or (I-2). The resist material may optionally contain an organic solvent.

[3-1. Organic Solvent]

The organic solvent used in this embodiment is not particularly limited, and a known and commonly used one can be used. The organic solvent is preferably an organic solvent capable of uniformly dissolving or dispersing the metatungstic acid salt compound (I-1) or (I-2).

Examples of the organic solvent include a polar solvent, such as an alcohol solvent, an ether solvent, a ketone solvent, an amide solvent, an ester solvent, a nitrile solvent, or an aprotic polar solvent, and a nonpolar solvent, such as a hydrocarbon solvent. These organic solvents may be used alone or in combination of two or more types thereof.

Specific examples of the polar solvent include an alcohol solvent, such as methanol, ethanol, isopropyl alcohol (IPA), 3-methoxybutanol, 3-methyl-3-methoxybutanol, 1-methoxy-2-propanol, 4-methyl-2-pentanol, ethylene glycol, diethylene glycol, propylene glycol, dipropylene glycol, or propylene glycol monomethyl ether (PGME);

an ether solvent, such as diethyl ether, dipropyl ether, dibutyl ether, diisoamyl ether, tetrahydrofuran, anisole, propylene glycol monoethyl ether, ethylene glycol monomethyl ether, or ethylene glycol monoethyl ether;
a ketone solvent, such as acetone, methyl ethyl ketone, cyclopentanone, cyclohexanone, methyl n-pentyl ketone, methyl isopentyl ketone, 2-heptanone, acetophenone, propylene carbonate, or furfural;
an amide solvent, such as N,N-dimethylformamide, N,N-diethylformamide, acetamide, N-methylpyrrolidone, 1-ethyl-2-pyrrolidone, or 1-butyl-2-pyrrolidone;
an ester solvent, such as methyl lactate, ethyl lactate (EL), methyl acetate, ethyl acetate, methyl pyruvate, ethyl pyruvate, methyl methoxypropionate, or propylene glycol monomethyl ether acetate (PGMEA);
a nitrile solvent, such as acetonitrile, propionitrile, or benzonitrile; and
an aprotic polar solvent, such as γ-butyrolactone, δ-valerolactone, γ-lactam, δ-lactam, dimethyl sulfoxide (DMSO), sulfolane, 1,3-dimethyl-2-imidazolidinone, or tetramethylurea; and
the nonpolar solvent may be a hydrocarbon solvent, such as n-pentane, n-hexane, toluene, or xylene;
or the like.

These organic solvents may be used alone or in combination of two or more types thereof.

The organic solvent to be contained in the resist material according to the present embodiment is preferably a polar solvent from the perspective of coatability, more preferably an amide solvent, an ester solvent, an alcohol solvent, or a ketone solvent from the perspective of solubility. The amide solvent is preferably at least one selected from the group consisting of N,N-dimethylformamide, N,N-diethylformamide, acetamide, and N-methylpyrrolidone. Furthermore, from

the perspective of solubility, the ester solvent or the alcohol solvent preferably has an ester bond and/or a hydroxy group, and among those described above, at least one selected from the group consisting of propylene glycol monomethyl ether, methyl lactate, ethyl lactate, methyl acetate, ethyl acetate, methyl pyruvate, ethyl pyruvate, and propylene glycol monomethyl ether acetate is preferred.

When the organic solvent contains both an amide-based solvent and an ester-based solvent, the compounding ratio (mass ratio) between the amide-based solvent and the ester-based solvent is preferably 1:99 to 40:60, more preferably 5:95 to 20:80.

The organic solvent content of the resist material is not particularly limited and can be appropriately determined according to the method for applying the resist material to a substrate or the like, the thickness of the film to be applied, and the like. The organic solvent content of the resist material is such that the solid concentration of the resist material preferably ranges from 0.01% to 20% by mass, more preferably 0.1% to 15% by mass, still more preferably 0.2% to 10% by mass.

[3-2. Others]

If desired, the resist material according to the present embodiment can appropriately contain, as a miscible additive agent, for example, an additional resin for improving the performance of a resist film, a dissolution inhibitor, a plasticizer, a stabilizer, a colorant, an antihalation agent, a dye, or the like.

The resist material according to the present embodiment preferably does not contain an epoxy resin. Furthermore, hydrogen peroxide is also preferably not contained.

[4. Pattern Forming Method]

The pattern forming method according to the present embodiment includes a step of applying a resist material to a substrate, a step of exposing a resist film formed by the application step to light, and a step of developing the exposed resist film.

[4-1. Application Step]

The pattern forming method according to the present embodiment includes the step of applying a resist material to a substrate.

<Substrate>

The substrate used in the present embodiment is not particularly limited and can be a known and commonly used substrate. The substrate is, for example, a substrate for an electronic component, a substrate on which a predetermined wiring pattern is formed, or the like

The material of the substrate is, for example, but not limited to, a silicon wafer, a substrate made of a metal, such as copper, chromium, iron, or aluminum, a substrate made of an inorganic substance, such as glass, titanium oxide, or silicon dioxide, or the like.

The substrate may have any size, shape, and the like, may have a smooth, curved, or rough surface, and may be a flaky substrate or the like.

The surface of the substrate may be subjected to a surface treatment as necessary. In the case of a substrate having a hydroxy group on a surface layer thereof, the surface of the substrate may be treated with a silane coupling agent capable of reacting with the hydroxy group to change the surface layer of the substrate from hydrophilic to hydrophobic, thereby enhancing the adhesion between the substrate and a film containing a metal compound. The silane coupling agent is, for example, hexamethyldisilazane (HMDS) or the like.

<Application Method>

The method for applying the resist material to a substrate is not particularly limited and can be a known and commonly used method. The application method includes a dry method, for example, a chemical vapor deposition (CVD) method, such as thermal CVD, plasma CVD, or photo-CVD; a physical vapor deposition (PVD) method, such as vacuum deposition, plasma-assisted vapor deposition, sputtering, or ion plating; or the like. A wet method, for example, a coating method, such as spin coating, bar coating, roll coating, flow coating, dip coating, spray coating, ink jet printing, or screen printing, can also be used.

From the perspective of forming a film with a uniform thickness or the like, the method for applying the resist material according to the present embodiment to a substrate is preferably a wet method, such as a spin coating method or

screen printing, more preferably a spin coating method. After the coating film is formed, a backside rinse step, an edge bead removal step, or the like may be performed to remove an edge bead.

A resist film formed by applying the resist material to a substrate may be dried by any method, for example, using a heater (post-apply bake (PAB)), such as a hot plate, or a pressure reduction apparatus.

The baking conditions are not particularly limited and can be appropriately determined according to the type, application, and the like of the resist film. The baking temperature preferably ranges from 80°C to 300°C, more preferably 150°C to 250°C, still more preferably 170°C to 230°C. The baking time preferably ranges from 10 seconds to 300 seconds, more preferably 20 seconds to 180 seconds, still more preferably 30 seconds to 120 seconds.

The thickness of the resist film after drying is preferably, but not limited to, in the range of 0.5 to 100 nm, more preferably 1 to 75 nm, still more preferably 1 to 60 nm.

[4-2. Exposure Step]

The pattern forming method according to the present embodiment includes a step of exposing the resist film formed by the application step to light.

As an exposure apparatus in the exposure step of the resist film, for example, an ArF exposure apparatus, an electron-beam lithography system, an EUV exposure apparatus, or the like can be used. Furthermore, in the exposure step, exposure may be performed through a mask (mask pattern) on which a predetermined pattern is formed, or selective exposure may be performed by drawing or the like by direct irradiation with an electron beam without using a mask pattern.

The exposure wavelength is not particularly limited, and radiation, such as an ArF excimer laser (wavelength: 193 nm), a KrF excimer laser (248 nm), an $F_2$ excimer laser (wavelength: 157 nm), extreme ultraviolet (EUV), vacuum ultraviolet (VUV), an electron beam (EB), X-rays, soft X-rays, or the like may be used.

For an ArF excimer laser or a KrF excimer laser, the exposure level on the resist film preferably ranges from 1 to 200 $mJ/cm^2$, more preferably 20 to 60 $mJ/cm^2$. Furthermore, for extreme ultraviolet, the exposure level is, for example, 500 $mJ/cm^2$ or less, preferably 0.1 to 200 $mJ/cm^2$, more preferably 3 to 100 $mJ/cm^2$, still more preferably 5 to 50 $mJ/cm^2$.

For an electron beam, exposure at a dose in the range of 3 $\mu C/cm^2$ to 2 $mC/cm^2$ at 50 kV is preferred, and exposure at a dose in the range of 10 $\mu C/cm^2$ to 1.5 $mC/cm^2$ is more preferred.

After the resist film is exposed to light, a baking (post-exposure bake (PEB)) treatment may or may not be performed. The baking conditions are not particularly limited and can be appropriately determined according to the type, application, and the like of the resist film. The baking temperature preferably ranges from 80°C to 300°C, more preferably 150°C to 250°C, still more preferably 170°C to 230°C, using a heater, such as a hot plate. The baking time preferably ranges from 10 seconds to 300 seconds, more preferably 20 seconds to 180 seconds, still more preferably 30 seconds to 120 seconds.

[4-3. Development Step]

The pattern forming method according to the present embodiment includes the step of developing the exposed resist film. In the development step, the exposed resist film can be developed with a developer to form a pattern. A case where the exposed region remains as a pattern after development is a negative patterning process, and a case where the exposed region is removed after development is a positive patterning process. Whether a positive or negative pattern is formed can be appropriately selected depending on the resist material or the developer. Furthermore, the development may be followed by washing with a rinse liquid and drying, and in some cases, further followed by a baking treatment.

<Developer>

The developer used in the present embodiment is, for example, a developer containing at least one of a polar solvent, such as a ketone solvent, an ester solvent, an alcohol solvent, a nitrile solvent, an amide solvent, or an ether solvent, and an organic solvent, such as a hydrocarbon solvent.

Specific examples of the organic solvent include:

a ketone solvent, such as acetone, methyl ethyl ketone, cyclopentanone, cyclohexanone, methyl n-pentyl ketone, methyl isopentyl ketone, 2-heptanone, acetophenone, propylene carbonate, or furfural;
an ester solvent, such as methyl lactate, ethyl lactate (EL), methyl acetate, ethyl acetate, methyl pyruvate, ethyl pyruvate, methyl methoxypropionate, or propylene glycol monomethyl ether acetate (PGMEA);
an alcohol solvent, such as methanol, ethanol, isopropyl alcohol (IPA), 3-methoxybutanol, 3-methyl-3-methoxybutanol, 1-methoxy-2-propanol, 4-methyl-2-pentanol, ethylene glycol, diethylene glycol, propylene glycol, dipropylene glycol, or propylene glycol monomethyl ether (PGME);
a nitrile solvent, such as acetonitrile, propionitrile, or benzonitrile;

an amide solvent, such as N,N-dimethylformamide, N,N-diethylformamide, acetamide, N-methylpyrrolidone, 1-ethyl-2-pyrrolidone, or 1-butyl-2-pyrrolidone;

an ether solvent, such as diethyl ether, dipropyl ether, dibutyl ether, diisoamyl ether, tetrahydrofuran, anisole, propylene glycol monoethyl ether, ethylene glycol monomethyl ether, or ethylene glycol monoethyl ether; and

a hydrocarbon solvent, such as n-pentane, n-hexane, toluene, or xylene.

These may be used alone or in combination of two or more types thereof.

Among these, the organic solvent used in the developer is preferably a polar solvent, preferably contains an amide solvent, an alcohol solvent, or an ester solvent, more preferably contains an amide solvent or an ester solvent and an alcohol solvent.

The amide solvent is preferably at least one selected from the group consisting of N,N-dimethylformamide, N,N-diethylformamide, acetamide, and N-methylpyrrolidone.

Furthermore, the ester solvent is preferably one or more selected from the group consisting of methyl lactate, ethyl lactate (EL), methyl acetate, ethyl acetate, methyl pyruvate, ethyl pyruvate, methyl methoxypropionate, and propylene glycol monomethyl ether acetate.

The alcohol solvent is preferably at least one selected from the group consisting of ethanol, isopropyl alcohol (IPA), 3-methoxybutanol, 3-methyl-3-methoxybutanol, 1-methoxy-2-propanol, 4-methyl-2-pentanol, benzyl alcohol, and 4-methylbenzyl alcohol.

In a case where the developer contains both an amide solvent or an ester solvent and an alcohol solvent, the blend ratio (weight ratio) of the amide solvent or the ester solvent to the alcohol solvent preferably ranges from 5:95 to 95:5, more preferably 10:90 to 90:10.

The method for developing the exposed resist film using the developer is not particularly limited and can be a known and commonly used method. The method of developing using the developer is, for example, a method of immersing the substrate having the exposed resist film in the developer for a certain period (dip method), a method of spraying the developer on the surface of the exposed resist film (spray method), a method of discharging the developer from a discharge nozzle at a constant speed toward the surface of the exposed resist film on the substrate rotating at a constant speed (dynamic dispense method), or the like.

<Rinse Liquid>

The development with the developer may be followed by a step of washing with a rinse liquid. The rinse liquid is not particularly limited and may be a known and commonly used rinse liquid. As the rinse liquid, among the organic solvent(s) in the developer, a solvent that is less likely to dissolve the resist pattern can be appropriately selected and used.

The rinse liquid is, for example, but not limited to, at least one organic solvent selected from the group consisting of a hydrocarbon solvent, a ketone solvent, an ester solvent, an alcohol solvent, an amide solvent, and an ether solvent. Among these, the rinse liquid is preferably at least one organic solvent selected from the group consisting of a hydrocarbon solvent, a ketone solvent, an ester solvent, an alcohol solvent, and an amide solvent, more preferably at least one selected from the group consisting of an alcohol solvent and an ester solvent, still more preferably at least one alcohol solvent.

The alcohol solvent used in the rinse liquid is preferably a monohydric alcohol with 2 to 8 carbon atoms, and the monohydric alcohol may be linear, branched, or cyclic. Specific examples of the monohydric alcohol include ethanol, isopropyl alcohol(IPA), butanol, 1-hexanol, 1-heptanol, 1-octanol, 2-hexanol, 2-heptanol, 2-octanol, 3-hexanol, 3-heptanol, 3-octanol, 4-octanol, and benzyl alcohol.

These organic solvents may be used alone or in combination of two or more types thereof.

In addition to the organic solvents described above, the rinse liquid may further include an organic solvent other than the organic solvents described above or water. In a case where the rinse liquid contains water, the water content is preferably 30% or less by mass, more preferably 10% or less by mass, still more preferably 5% or less by mass, particularly preferably 3% or less by mass, relative to the total amount of the rinse liquid.

The method for washing the resist pattern with the rinse liquid is not particularly limited and can be a known and commonly used method. The method of washing using the rinse liquid is, for example, a method of immersing the resist pattern in the rinse liquid for a certain period (dip method), a method of spraying the rinse liquid onto the surface of the resist pattern (spray method), and a method of discharging the rinse liquid from a discharge nozzle at a constant speed toward the surface of the resist pattern rotating at a constant speed (dynamic dispense method), or the like.

[5. Patterned Structure]

A patterned structure according to the present embodiment is produced by forming a pattern of the resist material on a substrate by the pattern forming method.

The average thickness of part or all of the patterned structure according to the present embodiment is not

particularly limited and can be appropriately determined depending on the intended use or the like. The patterned structure preferably has a portion with an average thickness in the range of 10 to 100 nm, more preferably 15 to 75 nm. It is more preferable to have a portion with an average thickness in the range of 20 to 60 nm.

The pitch of part or all of the patterned structure according to the present embodiment is not particularly limited and can be appropriately determined depending on the intended use or the like. The patterned structure preferably has a portion with a pitch of 100 nm or less, more preferably has a portion with a pitch of 50 nm or less, and still more preferably has a portion with a pitch of 20 nm or less.

The resist material according to the present embodiment can be patterned by the pattern forming method to form a portion with a pitch of 50 nm, a portion with a pitch of 20 nm, and a portion with a pitch of 15 nm in part or all of the patterned structure.

[6. Resist Material for Electron Beam or EUV]

The resist material according to the present embodiment has high sensitivity to an electron beam and an EUV light source and therefore can be suitably used as a resist material for these exposure light sources.

## EXAMPLES

The present invention will be more specifically described below with reference to examples, but the present invention is not limited to these examples.

[1. Synthesis of Metatungstate Compounds]

<Synthesis Example 1>

10 g of water and 10 g of 1 mol/L aqueous hydrochloric acid were added to 16.67 g of ammonium metatungstate (manufactured by Nippon Inorganic Colour & Chemical Co., Ltd.: ammonium metatungstate hydrate ($(NH_4)_6[H_2W_{12}O_{40}]\cdot xH_2O$) and were stirred at room temperature for 60 minutes. An aqueous solution of 13.47 g of bis(2-trifluoromethylphenyl)phenylsulfonium chloride in 200 g of pure water was then added to this reaction liquid and was stirred at room temperature for 30 minutes. A powder prepared by filtering the reaction liquid was dried under reduced pressure at room temperature for 18 hours. The dried powder was dissolved in 100 g of dimethylformamide, 335 g of methanol was then added thereto, and a target compound was produced by crystallization at room temperature.

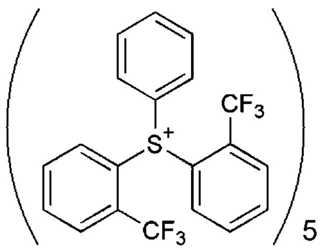
$[H_3W_{12}O_{40}]^{5-}$

$^1$H-NMR (400 MHz, DMSO-D6): δ (ppm) =6.77(s, 0.60H), 7.65-8.35(m, 13H). $^{183}$W-NMR (20.84 MHz, DMSO-D6): δ (ppm) = -87.59(s, 12W).
ESI-MS: POSITIVE m/z 399.1 ($[C_{20}H_{13}F_6S]^+$)
NEGATIVE m/z 712.3 (median) ($[H_4W_{12}O_{40}]^{4-}$)

<Synthesis Examples 2 to 8 and Comparative Synthesis Examples 1 and 2>

In Synthesis Examples 2 to 8 and Comparative Synthesis Examples 1 and 2, metatungstic acid salt compounds were synthesized in substantially the same manner as in Synthesis Example 1 described above, except that, in Synthesis Example 1, the type, concentration and amount of the acid, the amount of water added, the type and amount of the onium salt, and the amount of pure water added were changed as shown in Table 1.

The $^1$H-NMR, $^{183}$W-NMR, and ESI-MS of the hexakis(bis(2-trifluoromethylphenyl)phenylsulfonium) metatungstate obtained in Comparative Synthesis Example 1 are shown below.

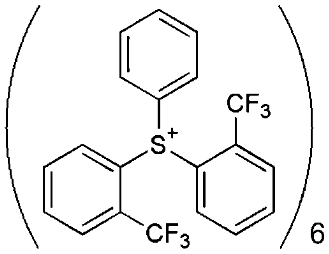 $[H_2W_{12}O_{40}]^{6-}$

$^1$H-NMR (400 MHz, DMSO-D6): δ (ppm) =5.97(s, 0.33H), 7.65-8.35(m, 13H). $^{183}$W-NMR (20.84 MHz, DMSO-D6): δ (ppm) = -100.13(s, 12W).
ESI-MS: POSITIVE m/z 399.1 ($[C_{20}H_{13}F_6S]^+$)
NEGATIVE m/z 712.3 (median) ($[H_4W_{12}O_{40}]^{4-}$)

The onium salts (1) and (2) shown in Table 1 are as follows.

· Onium salt (1): bis(2-trifluoromethylphenyl)phenylsulfonium chloride
· Onium salt (2): bis(4-tert-butylphenyl)iodonium sulfonate (MeOH solution)

In Synthesis Examples 1 to 8 and Comparative Synthesis Examples 1 and 2, the formation ratio between a metatungstic acid salt compound containing 5 onium cations and a metatungstic acid salt compound containing 6 onium cations was determined by comparing the integral value of a peak in the vicinity of 6.77 ppm (a proton peak attributable to $[H_3W_{12}O_{40}]^{5-}$) in $^1$H-NMR with the integral value of a peak in the vicinity of 5.97 ppm (a proton peak attributable to $[H_2W_{12}O_{40}]^{6-}$).
The results are shown in the column "Ratio" in Table 1.

[Table 1]

| | Acid | | | Water | pH | Onium salt | | Pure water | Ratio | |
|---|---|---|---|---|---|---|---|---|---|---|
| | Type | Concentration | Amount added (g) | Amount added (g) | | Type | Amount added (g) | Amount added (g) | $[H_3W_{12}O_{40}]^{5-}$ | $[H_2W_{12}O_{40}]^{6-}$ |
| Synthesis example 1 | Hydrochloric acid | 1 mol/L | 10 | 90 | 1.3 | Onium salt (1) | 13.47 | 200 | 100 | 0 |
| Synthesis example 2 | Sulfuric acid | 1 mol/L | 50 | 50 | 1.5 | Onium salt (1) | 13.47 | 200 | 100 | 0 |
| Synthesis example 3 | Nitric acid | 67 wt% | 50 | 50 | 1.6 | Onium salt (1) | 13.47 | 200 | 100 | 0 |
| Synthesis example 4 | Phosphoric acid | 1 mol/L | 50 | 50 | 1.7 | Onium salt (1) | 13.47 | 200 | 100 | 0 |
| Synthesis example 5 | Formic acid | 1 mol/L | 50 | 50 | 1.9 | Onium salt (1) | 13.47 | 200 | 100 | 0 |
| Synthesis example 6 | Hydrochloric acid | 1 mol/L | 50 | 98 | 2.0 | Onium salt (1) | 13.47 | 200 | 100 | 0 |
| Synthesis example 7 | Hydrochloric acid | 1 mol/L | 2 | 90 | 1.4 | Onium salt (2) | 15.64 | 200 | 100 | 0 |
| Synthesis example 8 | Hydrochloric acid | 1 mol/L | 0.3 | 99.7 | 2.8 5 | Onium salt (1) | 13.47 | 200 | 67 | 33 |
| Comparative synthesis example 1 | - | - | - | - | - | Onium salt (1) | 13.47 | 200 | 22 | 78 |
| Comparative synthesis example 2 | Hydrochloric acid | 1 mol/L | 0.1 | 99.9 | 3.1 | Onium salt (1) | 13.47 | 200 | 39 | 61 |

It can be seen from Table 1 that, in Synthesis Examples 1 to 8 in which an acidic solution having a pH of less than 3 was used, a metatungstic acid salt compound containing 5 onium cations could be selectively obtained. In addition, in Synthesis Examples 1 to 7, a metatungstic acid salt compound containing 6 onium cations was not present.

In contrast, in Comparative Synthesis Example 1 in which an acidic solution was not used, a metatungstic acid salt compound containing 6 onium cations was selectively obtained, and a metatungstic acid salt compound containing 5 onium cations was also contained. Even when an acidic solution was used, if the pH was higher than 3 as in Comparative Synthesis Example 2, a mixture of a metatungstic acid salt compound containing 5 onium cations and a metatungstic acid salt compound containing 6 onium cations was obtained, and the metatungstic acid salt compound containing 6 onium cations was contained in a larger proportion.

[2. Preparation of Resist Materials]

As resist materials, the metatungstic acid salt compounds obtained in Synthesis Examples 1 to 8 and Comparative Synthesis Examples 1 and 2 were compounded with dimethylformamide and ethyl lactate as organic solvents so as to have the contents shown in Table 2 to prepare resist materials R-1 to R-10.

[Table 2]

| Resist material | Metatungstic acid salt compound | | Organic solvent 1 | | Organic solvent 2 | |
|---|---|---|---|---|---|---|
| | Type | Amount added (g) | Type | Amount added (g) | Typ e | Amount added (g) |
| R-1 | Synthesis example 1 | 2.8 | DMF | 28.86 | EL | 68.34 |
| R-2 | Synthesis example 2 | 2.8 | DMF | 28.86 | EL | 68.34 |
| R-3 | Synthesis example 3 | 2.8 | DMF | 28.86 | EL | 68.34 |
| R-4 | Synthesis example 4 | 2.8 | DMF | 28.86 | EL | 68.34 |
| R-5 | Synthesis example 5 | 2.8 | DMF | 28.86 | EL | 68.34 |
| R-6 | Synthesis example 6 | 2.8 | DMF | 28.86 | EL | 68.34 |
| R-7 | Synthesis example 7 | 2.8 | DMF | 28.86 | EL | 68.34 |
| R-8 | Synthesis example 8 | 2.8 | DMF | 28.86 | EL | 68.34 |
| R-9 | Comparative synthesis example 1 | 2.8 | DMF | 28.86 | EL | 68.34 |
| R-10 | Comparative synthesis example 2 | 2.8 | DMF | 28.86 | EL | 68.34 |

[3. LS Pattern Formation]

The resist material R-1 to R-10 was applied using a spinner to a silicon wafer substrate, was subjected to a post-apply bake (PAB) treatment on a hot plate at a temperature of 200°C for 60 seconds, and was dried to form a resist film M-1 to M-9 with a thickness of 50 nm. The thickness of the formed resist film was measured with a thickness measuring apparatus ("M-2000D" manufactured by J. A. Woollam).

The resist films M-1 to M-10 were exposed through a mask using an EUV exposure apparatus while changing the exposure dose in a stepwise manner. After performing a PEB treatment at 180°C for 60 seconds, development was performed at 23°C for 60 seconds using a developer solution (DMF (10 parts by mass), IPA (90 parts by mass)), followed by rinsing with IPA for 60 seconds, to prepare a negative-tone 1:1 LS pattern having a line width of 50 nm. The optimum exposure dose Eop (mJ/cm$^2$) at which an LS pattern having a target size was formed was determined. The resist films M-1 to M-6 had an Eop of 100 mJ/cm$^2$, the resist film M-9 had an Eop of 115 mJ/cm$^2$, and the resist film M-10 had an Eop of 110 mJ/cm$^2$. For each of the resist films M-1 to M-10, a 1:1 LS pattern having a line width of 50 nm was prepared in the same manner as described above using the optimum exposure dose determined above. For the resist films M-7 and M-8, although the optimum exposure dose was not confirmed, an exposure dose of 100 mJ/cm$^2$ was used as the optimum exposure dose.

<Temporal Stability: CD Change Rate>

The prepared LS patterns were observed from the top of the LS patterns using a scanning electron microscope (trade name: S-9380) manufactured by Hitachi High-Technologies Corporation, and the line width (nm) was measured.

The change rate of pattern dimension (CD) of the line width of the prepared LS pattern relative to the mask pattern (CD change rate) was determined, and the temporal stability of the resist bottle was evaluated according to the following criteria. Table 3 shows the results.

$$\text{CD change rate} = (|\text{line width of LS pattern - mask pattern}|)/(\text{mask pattern}) \times 100$$

◦: the CD change rate is 5.0% or less
△: the CD change rate is more than 5.0% and 7.5% or less
×: the CD change rate is more than 7.5%

[Table 3]

| Resist film | Resist material | Eop (mJ/cm$^2$) | Mask pattern (nm) | LS pattern (nm) | CD change rate (%) | Evaluation |
|---|---|---|---|---|---|---|
| M-1 | R-1 | 100 | 50 | 49 | 2.0 | ○ |
| M-2 | R-2 | 100 | 50 | 49 | 2.0 | ○ |
| M-3 | R-3 | 100 | 50 | 49 | 2.0 | ○ |
| M-4 | R-4 | 100 | 50 | 48 | 4.0 | ○ |
| M-5 | R-5 | 100 | 50 | 48 | 4.0 | ○ |
| M-6 | R-6 | 100 | 50 | 49 | 2.0 | ○ |
| M-7 | R-7 | Unconfirmed | 50 | 48 | 4.0 | ○ |
| M-8 | R-8 | Unconfirmed | 50 | 47 | 6.0 | △ |
| M-9 | R-9 | 115 | 50 | 44 | 12.0 | × |
| M-10 | R-10 | 110 | 50 | 46 | 8.0 | × |

From Table 3, in the resist films M-9 and M-10 using the resist materials R-9 and R-10, which are mixtures of a metatungstic acid salt compound containing 5 onium cations and a metatungstic acid salt compound containing 6 onium cations and which contain the metatungstic acid salt compound containing 6 onium cations in a larger proportion, the CD change rates were large, specifically 12.0% and 8.0%, respectively.

In contrast, in the resist films R-1 to R-7 using the resist materials R-1 to R7, in which a metatungstic acid salt compound containing 6 onium cations is not present and which contain a metatungstic acid salt compound containing 5 onium cations, the CD change rates were less than 5.0%.

Furthermore, in the resist film M-8 using the resist material R-8, which is a mixture of a metatungstic acid salt compound containing 5 onium cations and a metatungstic acid salt compound containing 6 onium cations and which contains the metatungstic acid salt compound containing 5 onium cations in a larger proportion, the CD change rate was 6.0%, and a better result was obtained for the CD change rate compared with the resist films R-9 and R-10.

From the above, it is understood that a resist material containing a metatungstic acid salt compound synthesized by a method for selectively synthesizing a metatungstic acid compound containing 5 onium cations, as in Synthesis Examples 1 to 8, provides a favorable resist film.

## Claims

**1.** A method for producing a metatungstic acid salt compound represented by General Formula (I-1) or (I-2), the method comprising a step of performing salt exchange in an acidic solution having a pH of less than 3.

$$(A^+)_5 \cdot [H_3W_{12}O_{40}]^{5-} \qquad \text{(I-1)}$$

$$(A'^{2+})_5 \cdot ([H_3W_{12}O_{40}]^{5-})_2 \qquad \text{(I-2)}$$

[in Formulae (I-1) and (I-2),

each $A^+$ independently represents an onium cation; and
each $A'^{2+}$ independently represents an onium dication.]

2. The method for producing a metatungstic acid salt compound according to claim 1, wherein the onium cation is one or more selected from the group consisting of an organic sulfonium cation, an organic iodonium cation, and an organic ammonium cation.

3. The method for producing a metatungstic acid salt compound according to claim 1, wherein the acidic solution having a pH of less than 3 contains an inorganic acid, an organic acid having a carboxyl group, or an organic acid having a sulfo group.

4. The method for producing a metatungstic acid salt compound according to claim 1, wherein the salt exchange is salt exchange between a metatungstic acid salt represented by General Formula (II) and an onium salt represented by General Formula (III-1) or (III-2).

$$(B^+)_6 \cdot [H_2W_{12}O_{40}]^{6-} \quad \text{(II)}$$

$$A^+ \cdot X^- \quad \text{(III-1)}$$

$$A'^{2+} \cdot (X^-)_2 \quad \text{(III-2)}$$

[in Formula (II),

each $B^+$ independently represents $H^+$, a metal ion, or an ammonium ion;
in Formulae (III-1) and (III-2),
$A^+$ represents an onium cation;
$A'^{2+}$ represents an onium dication; and
$X^-$ represents a monovalent counter anion.]

5. A metatungstic acid salt compound for resists, the metatungstic acid salt compound being represented by General Formula (I-1) or (I-2).

$$(A^+)_5 \cdot [H_3W_{12}O_{40}]^{5-} \quad \text{(I-1)}$$

$$(A'^{2+})_5 \cdot ([H_3W_{12}O_{40}]^{5-})_2 \quad \text{(I-2)}$$

[in Formulae (I-1) and (I-2),

each $A^+$ independently represents an onium cation; and
each $A'^{2+}$ independently represents an onium dication.]

6. A resist material comprising the metatungstic acid salt for resists according to claim 5.

7. The resist material according to claim 6, further comprising a solvent.

8. The resist material according to claim 6 or 7, for electronic use or for EUV use.

9. A patterned structure formed using the resist material according to claim 6 or 7.

## INTERNATIONAL SEARCH REPORT

International application No. **PCT/JP2024/027411**

**A. CLASSIFICATION OF SUBJECT MATTER**

***C07C 381/12***(2006.01)i; ***C07F 11/00***(2006.01)i; ***G03F 7/004***(2006.01)i; ***G03F 7/038***(2006.01)i
FI: C07C381/12 CSP; G03F7/004 531; G03F7/038 505; C07F11/00 C

According to International Patent Classification (IPC) or to both national classification and IPC

**B. FIELDS SEARCHED**

Minimum documentation searched (classification system followed by classification symbols)

C07C381/12; C07F11/00; G03F7/004; G03F7/038

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | SPRANGERS, C R. et al. Where Are the Protons in α-[HxW12O40](8-x)- (x = 2-4)? Inorganic Chemistry. 2006, vol. 45, no. 24, pp. 9628-9630<br>abstract, supporting Information | 1-5 |
| Y | abstract, supporting Information | 6-9 |
| X | FUCHS, J. et al. Darstellung und Strukturuntersuchung von Polywolframaten Ein Beitrag zur Aufklarung des Parawolframations A / Preparation and Structure Investigation of Polytungstates A Contribution to the Paratungstate A Problem. Zeitschrift fur Naturforschung B. 1979, vol. 34, no. 3, pp. 412-422<br>abstract, p. 415, right column, line 18 | 5 |
| Y | abstract, p. 415, right column, line 18 | 6-9 |
| X | SEGUIN, L. et al. Structure of an hydrated tetrapropylamnonium polytungstate H3W12O40[N(C3H7)4]5 2H2O. European Journal of Solid State and Inorganic Chemistry. 1995, vol. 32, no. 3, pp. 181-194<br>abstract, conclusion | 5 |
| Y | abstract, conclusion | 6-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 October 2024** | **15 October 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
PCT/JP2024/027411

C. DOCUMENTS CONSIDERED TO BE RELEVANT

| Category* | Citation of document, with indication, where appropriate, of the relevant passages | Relevant to claim No. |
|---|---|---|
| Y | WO 2015/012177 A1 (JSR CORPORATION) 29 January 2015 (2015-01-29) claims, paragraphs [0067], [0076], examples | 6-9 |
| A | WO 2015/053194 A1 (NISSAN CHEMICAL INDUSTRIES, LTD.) 16 April 2015 (2015-04-16) claims, examples | 1-9 |
| A | JP 2013-040993 A (AZ ELECTRONIC MATERIALS IP LTD.) 28 February 2013 (2013-02-28) claims, examples | 1-9 |

Form PCT/ISA/210 (second sheet) (July 2022)

**INTERNATIONAL SEARCH REPORT**
**Information on patent family members**

International application No.
**PCT/JP2024/027411**

| Patent document cited in search report | Publication date (day/month/year) | Patent family member(s) | Publication date (day/month/year) |
|---|---|---|---|
| WO 2015/012177 A1 | 29 January 2015 | US 2016/0131978 A1<br>claims, paragraphs [0093]-[0095], [0108], examples | |
| WO 2015/053194 A1 | 16 April 2015 | US 2016/0251546 A1<br>claims, examples<br>CN 105612459 A<br>KR 10-2016-0065811 A | |
| JP 2013-040993 A | 28 February 2013 | US 2014/0356792 A1<br>claims, examples<br>KR 10-2014-0051995 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9725618 B
- US 10558119 B